# EUROPEAN PATENT APPLICATION

(11) **EP 4 036 242 A1**
(43) Date of publication of application: **03.08.2022**
(21) Application number: 20869176.6
(22) Date of filing: 23.09.2020
(51) Int. Cl.: C12N 15/90, C12N 15/85, A01K 67/027, C12Q 1/6888, C12N 15/11, C12N 5/10

(54) **METHOD FOR ESTABLISHING DIABETES DISEASE MODEL DOG**

(30) Priority: 23.09.2019 CN 201910901574
(71) Applicant: Beijing Sinogenetic Biotechnology Co., Ltd, Beijing 102604 (CN)
(72) Inventor: MI, Jidong, Beijing 102299 (CN); ZHAO, Jianping, Beijing 102299 (CN); ZHENG, Min, Beijing 102299 (CN)
(74) Representative: Kilger, Ute
(86) International application number: PCT/CN2020/117193
(87) International publication number: WO 2021/057806

(57) **Abstract**

Provided is a method for preparing a diabetic dog model by means of gene editing technology, a diabetic dog model prepared therefrom, as well as cells and issues thereof. The method comprises the following steps: (1) obtaining a dog fertilized egg cell, which comprises a point mutation in GCK gene, for a diabetic dog model by means of gene editing; and (2) transplanting the dog fertilized egg cell into one fallopian tube of a female dog, in which both fallopian tubes have been flushed, to prepare a diabetic dog model comprising a point mutation in GCK gene.

SEQUENCE LISTING

<110>  Beijing Sinogenetic Biotechnology Co., Ltd.

<120>  METHOD FOR ESTABLISHING DIABETES DISEASE MODEL DOG

<130>  B89503WOEP

<160>  11

<170>  PatentIn version 3.5

<210>  1
<211>  20
<212>  DNA
<213>  Canis lupus

<400>  1
ggatgcagag ggagatggcg                                                   20

<210>  2
<211>  20
<212>  DNA
<213>  Canis lupus

<400>  2
ggatgtagag ggagatggcg                                                   20

<210>  3
<211>  163
<212>  DNA
<213>  Canis lupus

<400>  3
gcagagcaga tcctggcgga cttccagatg caggaggcgg acttgaagaa ggtgatgcgg      60

cggatgtaga gggagatggc gcggggcctg cggctggaga cccacgagga ggccagcgtg     120

aagatgctgc ccacctacgt gcgctccacc ccagaaggct cag                       163

<210>  4
<211>  880
<212>  DNA
<213>  Canis lupus

<400>  4
gagctacaaa gggggagctg ccacctgcac cccccagcat gcctcggcca ctgcatttgg      60

gggctgctct tggaactctg ctgctctaac ttttcaactg gtcctaactg gtcatttgag     120

atgaggggtc atcttgggcc caggccagag ggcaaaggac actcccggtg tgtgcaggga     180

gggcctgact tggctgccac aggcccccag cccacccagc ttccccccac cccgtgcagg     240

cagagcagat cctggcggac ttccagatgc aggaggcgga cttgaagaag gtgatgcggc     300

ggatgtagag ggagatggcg cggggcctgc ggctggagac ccacgaggag gccagcgtga     360

agatgctgcc cacctacgtg cgctccaccc cagaaggctc aggtaccgcg tccctgggcc     420

ccagcaggga aggccagcct aggtccttgc agccatgggc tggttctcac ccgagctccg 480

tgccagcccc gccctggcat cttcaccgcc ttggggaaac tggctttgtg catcttgcaa 540

taggaaattt gttctcaggc tcagcccaat cctgaccctg cgctgtttca ctaggtcttt 600

ccttggaacc tgggcttgca gtggcttcag tccaaggcag cggctgccca ctgagctccc 660

cagttcacct ttgacctctt ccacagttga ctctggggtc ccctggtacc tggggtcagc 720

agccccctgt ccagctcctc tgatcccccc actccgtcct ctcctcctct tcctgatctt 780

ggggtcctgc tgggcagcac cccgaggcta atgcacttct cactgtgtct gcagaagtcg 840

gggacttcct ctccctggac ctgggcggca ccaacttcag 880

<210> 5
<211> 19
<212> DNA
<213> Artificial Sequence

<220>
<223> Artificial Sequence

<400> 5
ggtcatttga gatgagggg 19

<210> 6
<211> 19
<212> DNA
<213> Artificial Sequence

<220>
<223> Artificial Sequence

<400> 6
gaggaggaga ggacggagt 19

<210> 7
<211> 163
<212> DNA
<213> Canis lupus

<400> 7
gcagagcaga tcctggcgga cttccagatg caggaggcgg acttgaagaa ggtgatgcgg 60

cggatgcaga gggagatggc gcggggcctg cggctggaga cccacgagga ggccagcgtg 120

aagatgctgc ccacctacgt gcgctccacc ccagaaggct cag 163

<210> 8
<211> 25
<212> DNA
<213> Artificial Sequence

<220>
<223> Artificial Sequence

<400> 8

caccgggatg cagagggaga tggcg                                              25


<210>  9
<211>  25
<212>  DNA
<213>  Artificial Sequence

<220>
<223>  Artificial Sequence

<400>  9
aaaccgccat ctccctctgc atccc                                              25


<210>  10
<211>  37
<212>  DNA
<213>  Artificial Sequence

<220>
<223>  Artificial Sequence

<400>  10
aaggatgcaa caattgcatg tgagggccta tttccca                                 37


<210>  11
<211>  37
<212>  DNA
<213>  Artificial Sequence

<220>
<223>  Artificial Sequence

<400>  11
atgcaagtgc caattggcca tttgtctgca gaattgg                                 37

## Description

### TECHNICAL FIELD

The present disclosure relates to a method for preparing a diabetic dog model by means of gene editing, a diabetic dog model prepared therefrom, as well as the cells and tissues thereof.

### BACKGROUND

Dogs are one of the most commonly used experimental animals in fundamental medical researches and teaching. In particular, dogs play important role in experimental researches such as physiological, pharmacological and pathophysiological researches. Whole-genome sequencing of dogs identified about 19,300 genes, in which about 18,000 genes are identical to the known genes of human. Thus, dog genomic sequences are more similar to those of human than other experimental animals, such as mice. Dogs are also extremely similar to humans in terms of hereditary diseases. For example, more than 360 hereditary diseases, such as cancer, heart disease, partimutism, blindness and immune neurological diseases, are the same as in humans. In view of this, dogs are suitable for serving as model animals for the researches of human diseases. Furthermore, dogs have less hereditary diseases; good experimental reproducibility; well-developed blood circulation and nervous system; digestive system and internal organs similar to humans; and toxicological reactions similar to humans. Therefore, dogs are especially suitable for pharmacology, circulatory physiology, ophthalmology, toxicology, surgery and other researches. Further, dogs are docile and are easy to train, thereby being capable of cooperating well in experiments after short-term training. Therefore, dogs are recognized by international medical and biological researchers as ideal experimental animals.

At present, the methods for preparing dog models of diseases mainly include feeding, mechanical injury, immunization and other methods, which induce healthy animals to exhibit disease phenotypes. However, the dog models induced by these methods may have problems such as the inability to develop disease phenotypes, short duration of phenotypes, and the inability to simulate the symptoms of human disease. In contrast, the dog models obtained by gene knock-out or transgenic modification of the genome can exhibit the disease symptoms as primary symptoms, and have long-lasting and heritable disease phenotypes.

Diabetes is the third major disease after cardiovascular disease and cancer. So far, there are more than 400 million diabetic patients in the world, and more than 100 million diabetic patients in China. The prevalence of diabetes has exploded in the past two decades in China, due to the improvement of living standards, unhealthy diet and lack of exercise. In particular, type II diabetes account for 90 percent, and shows a trend of juvenile onset, thereby seriously affecting people's physical and mental health and quality of life. Type II diabetes is also called non-insulin dependent diabetes. Beta cells of type II diabetic patients can produce insulin, but are unable to respond to it, resulting in a relative lack of insulin. The factors inducing type II diabetes are numerous and complex.

Maturity-onset diabetes of the young (MODY) is one of the most common monogenic diabetes. MODY is caused by mutations in a gene associated with beta-cell function. In addition, MODY is autosomal dominantly inherited, develops before 25 years of age, and has multi-generational family history of diabetes. So far, at least 13 MODY forms (i.e., MODY1-13) have been discovered. Table 1 shows the related virulence gene and clinical molecular features. MODY1, MODY2, MODY3 and MODY5 are the most common. Maturity-onset diabetes of the young type 2 (MODY2) is caused by inactivating mutations in GCK gene. Glucokinase encoded by GCK gene catalyzes the conversion of glucose to glucose 6-phosphate. It is the first rate-limiting enzyme in the process of glucose metabolism. GCK is also a glucose sensor, and plays a key role in regulating the levels of blood glucose. MODY is characterized by, for example, dominant inheritance and early age of onset. One inactivating mutation in GCK gene can cause moderate hyperglycemia. Two inactivating mutations in GCK gene can cause severe hyperglycemia and show obvious symptoms after birth. GCK mutation(s) affect approximately 0.1% of the population, and account for a large proportion of juvenile diabetes. For the juvenile diabetes, most are caused by one mutation in GCK gene, which accounts for 10-60% of MODY patients. So far, 620 mutations have been found in 1,441 families, most of which come from England, France and Spain. MODY2 is also the most common form in Japan, and has less incidence rate in China.

MODY2 is a common form of juvenile diabetes, and generally shows clinical symptoms before the age of 25. GCK gene generally comprises one mutation, rarely comprises double mutations. The mutation(s), which cause clinical symptoms, basically occur in conserved regions. At present, patients with MODY2 are mainly treated with insulin injection. No other treatments have been reported. In addition, MODY2 is often misdiagnosed in the initial diagnosis, because it has clinical symptoms similar to type I diabetes. Thus, it is of great clinical significance to make further research on the treatment of MODY2.

However, research on the pathogenesis of MODY2 is very limited. There are still many unsolved questions: (1) whether the defects caused by GCK gene mutation(s) can be made up by upregulating the expression of hexokinase in glucose metabolism; (2) whether the hyperglycemia of patients with MODY2 is caused by GCK gene mutation(s) in liver or in β cells; (3) whether GCK gene deletion in other tissues and organs affects blood glucose level; and (4) whether stem cell therapy or gene repair therapy is effective for MODY2 patients. For this, animal models of diabetes need to be prepared first, in order to answer these questions.

In 1995, Andrew Grupe et al. prepared a GCK knock-out mouse model, and proved that the glucokinase secreted by pancreas β cells played a key role in maintaining the balance of blood glucose levels. Although they have great advantages, the mouse models have defects, for example, a distant relationship with humans, short lifespan, small body sizes, and quite different physiological and anatomical characteristics with respect to humans. Thus, the mouse models are difficult to accurately simulate the occurrence and treatment process of diseases. Large animal models can simulate human diseases more accurately as compared to mice. However, there is still a lack of large animal models of MODY2 diseases. This, to some extent, limits the research on stem cell therapy, gene therapy and drug screening for MODY2. Dogs are the best animals for preclinical drug trial and ideal model animals. The establishment of MODY2 diabetic dog models can provide animal models for stem cell therapy, gene repair therapy and drug screening of MODY2 diabetes, thereby providing an important preclinical trial basis for the clinical treatment of human MODY2 diabetes. It will be of great significance for the treatment and research of human MODY2 diabetes. Therefore, it is significant to prepare SHANK3 knock-out model dogs by means of the latest genome editing technology.

### SUMMARY

The present disclosure provides a method for preparing a diabetic dog model comprising point mutation(s) in GCK gene by means of gene editing. The method comprises: selecting a targeting site directed to dog GCK gene; preparing a BE3-expressing vector, which is proven to be effective and then subjected to *in vitro* transcription to obtain mRNA; intracytoplasmically injecting the mRNA into a dog fertilized egg cell; transplanting the embryo into the body of a female dog, in which both fallopian tubes have been flushed, to prepare a diabetic dog model comprising point mutation(s) in GCK gene. The present disclosure also relates to the GCK knock-out diabetic model prepared therefrom, as well as the cells and tissues thereof.

In the first aspect, the present disclosure provides a method for preparing a diabetic dog model comprising point mutation(s) in GCK gene, which comprises the following steps: (1) obtaining a dog fertilized egg cell, which comprises point mutation(s) in GCK gene, for a diabetic dog model by means of gene editing; and (2) transplanting the dog fertilized egg cell into one fallopian tube of a female dog, in which both fallopian tubes have been flushed, to prepare a diabetic dog model comprising point mutation(s) in GCK gene.

The gene editing in step (1) comprises BE3, CRISPR/Cas9, TALEN and ZFN.

In the second aspect, the present disclosure provides a method for preparing a diabetic dog model comprising point mutation(s) in GCK gene, which comprises the following steps: (1) determining a targeting site directed to exon 2 based on the sequence of dog GCK gene; (2) synthesizing an sgRNA sequence based on the targeting site determined in step (1), and ligating the synthesized sgRNA sequence into a backbone vector to construct an sgRNA targeting vector; (3) obtaining sgRNA with *in vitro* transcription of the sgRNA targeting vector, and obtaining BE3 mRNA with *in vitro* transcription; (4) mixing the sgRNA and BE3 mRNA obtained in step (3) and intracytoplasmically injecting into a dog fertilized egg cell; and (5) transplanting the dog fertilized egg cell into one fallopian tube of a female dog, in which both fallopian tubes have been flushed, to prepare a diabetic dog model comprising point mutation(s) in GCK gene.

Preferably, the targeting site is determined based on the sequence of exon 2 of GCK gene.

Preferably, the exon 2 of GCK gene is modified with, for example, insertion, deletion, substitution, and/or addition.

Preferably, the exon 2 of GCK gene is modified with point mutation.

Preferably, one targeting site is designed based on the sequence of GCK gene, and comprise a sequence of 5'-GGATGCAGAGGGAGATGGCG-3' (SEQ ID NO: 1).

Preferably, the sgRNA sequence synthesized based on the targeting site and its complementary sequence comprise, respectively:
F: CACCGggatgcagagggagatggcg (SEQ ID NO:8); and
R: AAACcgccatctccctctgcatccC (SEQ ID NO:9).

Preferably, the backbone vector is T7-gRNA commercially available from Addgene company.

Preferably, in step (5), the dog fertilized egg cell is transplanted into one fallopian tube with less bleeding of the female dog, in which both fallopian tubes have been flushed.

In the third aspect, the present disclosure provides a method for preparing a diabetic dog model comprising point mutation(s) in GCK gene, which comprises the following steps:(1) determining a targeting site directed to exon 2 based on the sequence of dog GCK gene; (2) synthesizing an sgRNA sequence according to the targeting site determined in step (1), and ligating the synthesized sgRNA sequence into a backbone vector to construct an sgRNA targeting vector; (3) obtaining sgRNA with *in vitro* transcription of the sgRNA targeting vector, and obtaining BE3 mRNA with *in vitro* transcription; (4) mixing the sgRNA and BE3 mRNA obtained in step (3) and intracytoplasmically injecting into a dog somatic cell, and transplanting the nucleus of the somatic cell into a dog enucleated egg cell; and (5) transplanting the dog enucleated egg cell into one fallopian tube of a female dog, in which both fallopian tubes have been flushed, to prepare a diabetic dog model comprising point mutation(s) in GCK gene.

In the fourth aspect, the present disclosure provides a diabetic dog model comprising point mutation(s) in GCK gene. The targeting vector for point mutation of dog GCK gene comprises an sgRNA sequence, which is designed based on the targeting site in exon 2 of dog GCK gene; and a backbone vector.

Preferably, the exon is exon 2 of dog GCK gene. Preferably, the backbone vector is T7-gRNA commercially available from Addgene.

Preferably, the targeting site comprises a sequence of 5'-GGATGCAGAGGGAGATGGCG-3' (SEQ ID NO: 1) .

Preferably, the sgRNA sequence synthesized based on the targeting site and its complementary sequence comprises, respectively, the following sequences:
F: CACCGggatgcagagggagatggcg (SEQ ID NO: 8), and
R: AAACcgccatctccctctgcatccC (SEQ ID NO: 9).

In the fifth aspect, the present disclosure provides a somatic cell, tissue and organ of the diabetic dog model comprising point mutation(s) in GCK gene obtained by the method of any one of the first to third aspects.

Preferably, the somatic cell, tissue and organ comprise the following sequence:
5'-GGATGTAGAGGGAGATGGCG-3' (SEQ ID NO: 2).

Preferably, the somatic cell, tissue and organ comprise the following sequence:

GCAGAGCAGATCCTGGCGGACTTCCAGATGCAGGAGGCGGACTTGAAGAAGGT
GATGCGGCGGATGTAGAGGGAGATGGCGCGGGGCCTGCGGCTGGAGACCCAC
GAGGAGGCCAGCGTGAAGATGCTGCCCACCTACGTGCGCTCCACCCCAGAAG
GCTCAG (SEQ ID NO: 3).

Preferably, the somatic cell, tissue and organ comprise the following sequence:

GAGCTACAAAGGGGGAGCTGCCACCTGCACCCCCCAGCATGCCTCGGCCACT
GCATTTGGGGGCTGCTCTTGGAACTCTGCTGCTCTAACTTTTCAACTGGTCCTA
ACTGGTCATTTGAGATGAGGGGTCATCTTGGGCCCAGGCCAGAGGGCAAAGGA
CACTCCCGGTGTGTGCAGGGAGGGCCTGACTTGGCTGCCACAGGCCCCCAGC
CCACCCAGCTTCCCCCCACCCCGTGCAGGCAGAGCAGATCCTGGCGGACTTCC
AGATGCAGGAGGCGGACTTGAAGAAGGTGATGCGGCGGATGTAGAGGGAGAT
GGCGCGGGGCCTGCGGCTGGAGACCCACGAGGAGGCCAGCGTGAAGATGCT
GCCCACCTACGTGCGCTCCACCCCAGAAGGCTCAGGTACCGCGTCCCTGGGC
CCCAGCAGGGAAGGCCAGCCTAGGTCCTTGCAGCCATGGGCTGGTTCTCACCC
GAGCTCCGTGCCAGCCCCGCCCTGGCATCTTCACCGCCTTGGGGAAACTGGCT
TTGTGCATCTTGCAATAGGAAATTTGTTCTCAGGCTCAGCCCAATCCTGACCCTG
CGCTGTTTCACTAGGTCTTTCCTTGGAACCTGGGCTTGCAGTGGCTTCAGTCCA

AGGCAGCGGCTGCCCACTGAGCTCCCCAGTTCACCTTTGACCTCTTCCACAGTT
GACTCTGGGGTCCCCTGGTACCTGGGGTCAGCAGCCCCCTGTCCAGCTCCTCT
GATCCCCCCACTCCGTCCTCTCCTCCTCTTCCTGATCTTGGGGTCCTGCTGGGC
AGCACCCCGAGGCTAATGCACTTCTCACTGTGTCTGCAGAAGTCGGGGACTTC
CTCTCCCTGGACCTGGGCGGCACCAACTTCAG (SEQ ID NO:4).

Preferably, the somatic cell is classified as skin fibroblast GCK-KO-190619 of beagle, which is a diabetic dog model comprising point mutation(s) in GCK gene, and is deposited in China General Microbiological Culture Collection Center (CGMCC) located at No.3, Yard 1, Beichen West Road, Chaoyang District, Beijing (postal code 100101) on July 23, 2019 under CGMCC deposit No. 18305.

In the sixth aspect, the present disclosure provides a pair of primers for detecting a diabetic dog model comprising point mutation(s) in GCK gene, wherein the diabetic dog model comprises a genomic sequence including a sequence as shown by 5'-GGATGTAGA GGGAGATGGCG-3'(SEQ ID NO: 2). The pair of primers is designed based on the following sequence:

GAGCTACAAAGGGGGAGCTGCCACCTGCACCCCCCAGCATGCCTCGGCCACT
GCATTTGGGGGCTGCTCTTGGAACTCTGCTGCTCTAACTTTTCAACTGGTCCTA
ACTGGTCATTTGAGATGAGGGGTCATCTTGGGCCCAGGCCAGAGGGCAAAGGA
CACTCCCGGTGTGTGCAGGGAGGGCCTGACTTGGCTGCCACAGGCCCCCAGC
CCACCCAGCTTCCCCCCACCCCGTGCAGGCAGAGCAGATCCTGGCGGACTTCC
AGATGCAGGAGGCGGACTTGAAGAAGGTGATGCGGCGGATGTAGAGGGAGAT
GGCGCGGGGCCTGCGGCTGGAGACCCACGAGGAGGCCAGCGTGAAGATGCT
GCCCACCTACGTGCGCTCCACCCCAGAAGGCTCAGGTACCGCGTCCCTGGGC
CCCAGCAGGGAAGGCCAGCCTAGGTCCTTGCAGCCATGGGCTGGTTCTCACCC
GAGCTCCGTGCCAGCCCCGCCCTGGCATCTTCACCGCCTTGGGGAAACTGGCT
TTGTGCATCTTGCAATAGGAAATTTGTTCTCAGGCTCAGCCCAATCCTGACCCTG
CGCTGTTTCACTAGGTCTTTCCTTGGAACCTGGGCTTGCAGTGGCTTCAGTCCA
AGGCAGCGGCTGCCCACTGAGCTCCCCAGTTCACCTTTGACCTCTTCCACAGTT
GACTCTGGGGTCCCCTGGTACCTGGGGTCAGCAGCCCCCTGTCCAGCTCCTCT
GATCCCCCCACTCCGTCCTCTCCTCCTCTTCCTGATCTTGGGGTCCTGCTGGGC
AGCACCCCGAGGCTAATGCACTTCTCACTGTGTCTGCAGAAGTCGGGGACTTC
CTCTCCCTGGACCTGGGCGGCACCAACTTCAG (SEQ ID NO:4).

Preferably, the pair of primers comprise the following sequences:
GCK-CBE-S2-F: 5' GGTCATTTGAGATGAGGGG 3' (SEQ ID NO: 5);
GCK-CBE-S2-R: 5' GAGGAGGAGAGGACGGAGT 3' (SEQ ID NO: 6).

In the seventh aspect, the present disclosure provides a kit for detecting a diabetic dog model comprising point mutation(s) in GCK gene, wherein the diabetic dog model comprises a genomic sequence including a sequence as shown by GGATGTAGAGGGAGATGGCG (SEQ ID NO: 2), and the kit comprises a pair of primers which is designed based on the sequence as shown by SEQ ID NO: 4 or 3.

Preferably, the pair of primers comprises the following sequences:
GCK-CBE-S2-F: 5' GGTCATTTGAGATGAGGGG 3' (SEQ ID NO: 5);
GCK-CBE-S2-R: 5' GAGGAGGAGAGGACGGAGT3' (SEQ ID NO: 6).

In the eighth aspect, the present disclosure provides a diabetic dog model comprising point mutation(s) in GCK gene obtained by the method of any one of the first to third aspects.

The dog GCK gene comprises eight exons in total. The present disclosure comprises a step of performing gene targeting for exon 2.

The wild type exon 2 of GCK gene has the following sequence:

GCAGAGCAGATCCTGGCGGACTTCCAGATGCAGGAGGCGGACTTGAAGAAGGT
GATGCGGCGGATGCAGAGGGAGATGGCGCGGGGCCTGCGGCTGGAGACCCA
CGAGGAGGCCAGCGTGAAGATGCTGCCCACCTACGTGCGCTCCACCCCAGAA
GGCTC AG (SEQ ID NO: 7).

The exon 2 including a point mutation has the following sequence:

GCAGAGCAGATCCTGGCGGACTTCCAGATGCAGGAGGCGGACTTGAAGAA
GGTGATGCGGCGGATGTAGAGGGAGATGGCGCGGGGCCTGCGGCTGGAGAC
CCACGAGGAGGCCAGCGTGAAGATGCTGCCCACCTACGTGCGCTCCACCCCA
GAAGGCTCAG (SEQ ID NO: 3).

In addition to exon 2, a dog also can be induced to show diabetic phenotype by gene targeting at any sequence of other exons of dog GCK gene, thereby obtaining a diabetic dog model comprising point mutations GCK gene.

It is judged that a female dog is in proestrus when bloody secretions flow out of the vagina of the female dog. Then, blood is collected to determine the concentration of progesterone. When the concentration of progesterone reaches 4-7 ng/mL, it can be determined that the female dog is in ovulation. 48-72 hours after ovulation, oocytes can develop to second meiotic metaphase. To obtain prokaryotic embryo(s) at one-cell stage, the female dog need natural mating 48 hours after ovulation, and the fertilized embryo(s) will be removed by flushing after 18 hours of *in vivo* insemination.

When flushing the embryo(s), the ovary and the uterine-canal junction of uterus is firstly exposed, and a metal injection needle with rounded tip is inserted into the fimbriae of a fallopian tube at the rupture of ovarian follicle. Then, the needle and the fimbria of the fallopian tube are ligated and fixed. Then, a blood-taking needle is punctured into the fallopian tube at the uterine-canal junction of fallopian tube, and flushes the fallopian tube with 10 mL of HEPES buffered TCM199 medium (HM, GIBCO, 11150) containing 10% fetal bovine serum. The flushed fluid is discharged from the needle ligated with the fimbria of the fallopian tube, and collected into a 10 mL centrifugation tube. Both fallopian tubes are flushed in this way.

In the present disclosure, the targeting site is selected based on the exons of dog GCK gene sequence by means of gene editing. The sgRNA targeting vector and BE3-expressing vector are constructed based on the sequence of the targeting site and proven to be effective, and then are subjected to *in vitro* transcription to obtain mRNA. Then, the obtained mRNAs are intracytoplasmically injected into the dog fertilized egg cells. The dog fertilized egg cells are then transplanted into one fallopian tube of a female dog, in which both fallopian tubes have been flushed, to prepare a diabetic dog model comprising point mutation(s) in GCK gene. This is the first time to successfully obtain a diabetic dog model comprising point mutation(s) in GCK gene in the world.

Dogs have close relationship with humans, and live in the same environment with humans. Dogs also have great genetic similarity with humans and relatively simple genetics. Diabetes is a common endocrinopathy for dogs, and thus dogs can be an ideal model animal of diabetes. The present disclosure prepares diabetic dog models via point mutation of exon 2 of dog GCK gene to simulate the genetic defects of human diabetes. Such diabetic dog models prepared by this method are the first one in the world, which can effectively compensate for the defects of diabetic mice models. Meanwhile, the diabetic dog models of the present disclosure have high similarity with humans in genetic defects, and can be used for the research of new techniques for the treatment of human diabetes, the development of new drugs, and the research of the diabetic pathogenesis. Thus, the present disclosure can lay a foundation for promoting the research of diabetes, especially the screening of diabetic drugs.

### Definitions of abbreviations and key terms

ICI: intracytoplasmic injection, which refers to injecting gene(s) into the cytoplasm of a fertilized egg cell through micro-manipulation by using a micro-injection needle.

### BRIEF DESCRIPTION OF THE DRAWINGS

FIG. 1 shows the alignment result of the PCR products of the target site in a wild-type dog and a diabetic dog model comprising a point mutation in GCK gene. Wherein, 190619, 190627, 190628 and 190761 represent diabetic dog models comprising point mutation in GCK gene, and WT represents a wild-type dog.
FIG. 2 shows the alignment result of the PCR products of the target site of 190625 and 190626, which are littermates of 190627 and 190628, and a WT dog (non-littermate).
FIG. 3 shows the picture of a diabetic dog model prepared in the present disclosure, which comprises the point mutation in GCK gene. Wherein, the dog on left is diabetic dog model 190627 comprising the point mutation in GCK gene, and the dog on right is wild-type littermate 190626.
FIG. 4 shows that the blood glucose level of the mutant dog is significantly higher than that of the wild-type littermate.
FIG. 5 shows that the weight gain of the mutant dog is significantly slower than that of the wild-type littermate.

### DETAILED DESCRIPTION

Hereinafter, the technical solutions of the present disclosure will be further described with reference to the examples and drawings. These examples are merely used for illustrating the present invention, but not to limit the protection scope thereof.

### Examples

(1) Preparement of a diabetic dog model comprising a point mutation in GCK gene, which comprises the following steps:
   1) determining a targeting site directed to exon 2 based on the sequence of dog GCK gene;
   2) synthesizing an sgRNA sequence based on the targeting site determined in step (1), and ligating the synthesized sgRNA sequence into a backbone vector to construct an sgRNA targeting vector;
   3) obtaining sgRNA with *in vitro* transcription of the sgRNA targeting vector, and obtaining BE3 mRNA with *in vitro* transcription;
   4) transfecting beagle skin fibroblasts, screening out cell clones, extracting DNA from the cell clones, performing PCR by using targeting site-specific primers, sequencing the PCR products to obtain the gene mutation results, and then calculating the vector targeting efficiency;
   5) mixing the sgRNA and CBE3 mRNA obtained in step (3), and intracytoplasmically injecting into a dog fertilized egg cell; and
   6) transplanting the fertilized egg cell into one fallopian tube with less bleeding of a female dog, in which both fallopian tubes have been flushed.

A vector expressing sgRNA directed to exon 2 of dog GCK gene was designed based on the sequence of dog GCK gene. One targeting site is designed to achieve point mutation at the target site. The targeting site of this example has the following sequence: 5'-GGATGCAGAGGGAGATGGCG-3' (SEQ ID NO: 1).

In this example, the sgRNA synthesized in step 2) and its complementary sequence (including sticky ends after annealling) have, respectively, the following sequences:
F: CACCGggatgcagagggagatggcg (SEQ ID NO: 8) and
R: AAACcgccatctccctctgcatccC (SEQ ID NO: 9).

When constructing the vector, the synthesized targeting sequences were annealed and ligated into PX330 which had been digested with Bbsl. The sgRNA expression cassette was PCR amplified by using the following primers:
SS72: AAGGATGCAA caattg catgtgagggcctatttccca (SEQ ID NO: 10); and
SS73-2: ATGCAAGTGC caattg gccatttgtctgcagaattgg (SEQ ID NO: 11).
The PCR products were purified and recovered. Then, the PCR products and pCBE3 were digested with Mfel-HF, purified, recovered and ligated. The resulted vector was sequenced to confirm the sgRNA-expressing vector was constructed successfully. The sgRNA-expressing vector was transformed, amplified and extracted. The targeting site of PCBE3 plasmid was PCR amplified. Then, the PCR products were recovered and were subjected to *in vitro* transcription by using an *in vitro* transcription kit. The obtained mRNAs were diluted to required injection concentration, subpackaged, and stored at -80°C. The aliquoted Cas9 and gRNA mRNA directed to the targeting site were mixed in a volume ratio of 1:1, and then used for cytoplasmic injection of the fertilized egg cells.

In particular, linearize the pBE3-EGFP plasmid in a reaction system including 30 µg plasmid, 5 µL restriction enzyme Bsal, 10 µL 10×Buffer and ddH₂O, in a total volume of 100 µL; add 100 µL phenol: chloroform: isopropyl alcohol (25:24:1) to purify the linearized plasmid; centrifuge at 12,000g for 5min; remove 50 µL supernatant to a 1.5 mL centrifugation tube without Rnase; add 1/10 volume of sodium acetate and 3 volumes of anhydrous ethanol to precipitate plasmid DNA; centrifuge at 12,000g for 5min; discard the supernatant; remove and discard residual supernatant; add 150 µL 70% ethanol to wash the plasmid, centrifuge at 12,000g for 5min; dry in air for 3-5 min, dissolve DNA with 15 µL RNase-free ddH₂O; and measure the concentration of DNA.

The *in vitro* transcription of mRNA was performed with a kit (Ambion) in an *in vitro* transcription system which comprised 1µg linearized plasmid DNA, 10µL 2×NTP/CAP, 2µL 10×Buffer, 2µL RNA polymerase and ddH₂O, in a total volume of 20µL. After mixing well, incubate for 1 hour at 37°C, add 1 µL TURBO DNase and digest plasmid templates, and incubate for 30 min at 37°C; mix 20µL *in vitro* transcription products, 20 µL Poly(A) polymerase and nuclease-free ddH₂O to obtain a system of *in vitro* transcription mRNA plus poly(A) in a total volume of 100µL; incubate for 1 hour at 37°C; then, add 350µL binding buffer into the reaction system and mix well; then add 250 µL anhydrous ethanol and mix well; transfer into an mRNA purification column, centrifuge at 10,000g for 1min at room temperature; discard the filtrate, centrifuge the empty column for 1min to rinse off impurities such as proteins; place the column into a new centrifugation tube, add 50µL RNA eluent to the central position of the column, press the lid, incubate for 10 min at 65°C, then centrifuge at 10,000g for 1min at room temperature, and measure RNA quality and concentration. The CRISPR sgRNA and BE3 mRNA were mixed, resulting that the final concentration of sgRNA was 20 ng/µL and the final concentration of BE3 was 200ng/µL, which is used for cytoplasmic injection.

Dog skin fibroblasts were co-transfected with the constructed gRNA and BE3 plasmids, and screened with G418. DNA was extracted from cell clones obtained by screening, and used as a template for PCR amplifying, with primers GCK-CBE-S2-F (GGTCATTTGAGATGAGGGG, SEQ ID NO: 5) and GCK-CBE-S2-R (GAGGAGGAGAGGACGGAGT, SEQ ID NO: 6), a DNA fragment of 660 bp upstream and downstream of the target site recognized by the sgRNA. The targeting efficiency of the vectors was determined by sequencing of the target DNA fragment. The results showed that the point mutation efficiency of the target site was about 60% after transfection, screening and PCR product sequencing. Therefore, the above processes could be used for the preparement of a diabetic dog model comprising a point mutation in GCK gene.

10 female beagles with spontaneous estrous were used as donors of fertilized egg cells and receptors of embryo implantation for experimental research. Blood was collected from all female dogs to determine the progesterone levels in the blood. When the progesterone level reached 4-7 ng/mL, it was determined that the female dogs were in ovulation. 48 hours after ovulation, the female dogs were subjected to natural mating, followed by flushing the fertilized egg cells through surgical means. 53 fertilized egg cells were collected from 10 female dogs, treated with TCM199 medium containing 0.1% hyaluronidase to remove cumulus granulosa cells, put into HM microdroplets, and then placed on an inverted microscope equipped with a micromanipulator. A mixture containing sgRNA and Cas9 was sucked with a micro-injection needle, and injected into cytoplasm of the fertilized egg cells. After the intracytoplasmic injection, the egg cells were loaded into embryo transfer tubes, and were injected, from the fimbria, into one fallopian tube with less bleeding during the embryo flushing.

After puppies were born, ear and tail tissues were collected for identification. Cut the tissues into pieces in a centrifugation tube, add protease K and incubate in a water bath of 56°C for lysis for 1-3 hours. Add 700µL Genomic Lysis Buffer with a pipette into the lysis system, mix upside down, and then centrifuge at 10000g for 1min. Remove the supernatant to a purifying column with a pipette, stand for 1min at room temperature, and centrifuge at 10000g for 1min. Place the purifying column in a new collection tube, add 200µL DNA pre-wash buffer, stand for 1min at room temperature, centrifuge at 10000g for 1min, and discard effluent. Add 400µL g-DNA washing buffer to the column, stand for 1min at room temperature, centrifuge at 10000g and discard effluent. Re-centrifuge the purifying column and collection tube at 10000g for 2min. Place the purifying column in a new 1.5mL centrifugation tube, add 50µL elution buffer, stand for 2min at room temperature, and centrifuge at 12,000rpm for 1min to obtain a solution of dog genomic DNA.

The dog genomic DNA was used as a template to carry out PCR, with the primers GCK-CBE-S2-F: GGTCATTTGAGATGAGGGG (SEQ ID NO: 5) and GCK-CBE-S2-R: GAGGAGGAGAGGACGGAGT (SEQ ID NO: 6), to amplify a DNA fragment of 660bp upstream and downstream of the target site recognized by the sgRNA. The amplified fragment was subjected to DNA sequencing, and aligned with the sequence of dog GCK gene to determine the mutation type in GCK gene.

The sequencing and alignment results showed that 4 of 13 puppies had point mutation in the target site.

At 8:00 every morning, the blood glucose levels were measured simultaneously for the positive (mutant) and wild-type dogs. (1) Insert a test strip into a blood glucose meter (Sinocare); (2) stab the ear veins of a puppy with a blood lancet; (3) seep blood drop; (4) align the reaction zone of the test strip to the blood drop to syphon the blood; (5) keep still until the blood glucose meter beeps and shows blood glucose value. The body weight of the positive (mutant) and wild-type dogs were also measured at 8:00 every morning, simultaneously.

**Table 1: Embryo Transfer Summary**

| No | No. of receptor dogs | Number of transferred embryos | Number of litters | Number of dogs comprising point mutation in GCK gene |
|---|---|---|---|---|
| 1 | FRG1492 | 5 | 7 | 1 (190619) |
| 2 | FRG11482 | 7 | 4(190625/190626/1 90627/190628) | 2 (190627/190628) |
| 3 | FRG11171 | 7 | 0 | 0 |
| 4 | FRG1442 | 9 | 0 | 0 |
| 5 | FRG11484 | 4 | 0 | 0 |
| 6 | FRG1681 | 4 | 0 | 0 |
| 7 | FRG11493 | 8 | 0 | 0 |
| 8 | FRG11565 | 4 | 2 | 1 (190761) |
| 9 | FRG180103 | 2 | 0 | 0 |
| 10 | FRG180816 | 3 | 1 | 0 |
| Total: 10 | | 53 | 14 | 4 |

In Table 1, dog Nos. 190625 and 190626 were wild-type littermates of mutant dog Nos. 190627 and 190628.

**Table 2: Alignment of characteristic gene sequences of diabetic dog models comprising point mutation in GCK gene, wild-type littermates and wild-type dogs.**

| No. of dogs | Gene sequence | Gene type |
|---|---|---|
| 190619 | 5'-GGATGTAGAGGGAGATGGCG-3' | Mutant sequence |
| 190627 | 5'-GGATGTAGAGGGAGATGGCG-3' | Mutant sequence |
| 190628 | 5'-GGATGTAGAGGGAGATGGCG-3' | Mutant sequence |
| 190761 | 5'-GGATGTAGAGGGAGATGGCG-3' | Mutant sequence |
| 190625 | 5'-GGATGCAGAGGGAGATGGCG-3' | Wild-type sequence |
| 190626 | 5'-GGATGCAGAGGGAGATGGCG-3' | Wild-type sequence |
| WT | 5'-GGATGCAGAGGGAGATGGCG-3' | Wild-type sequence |

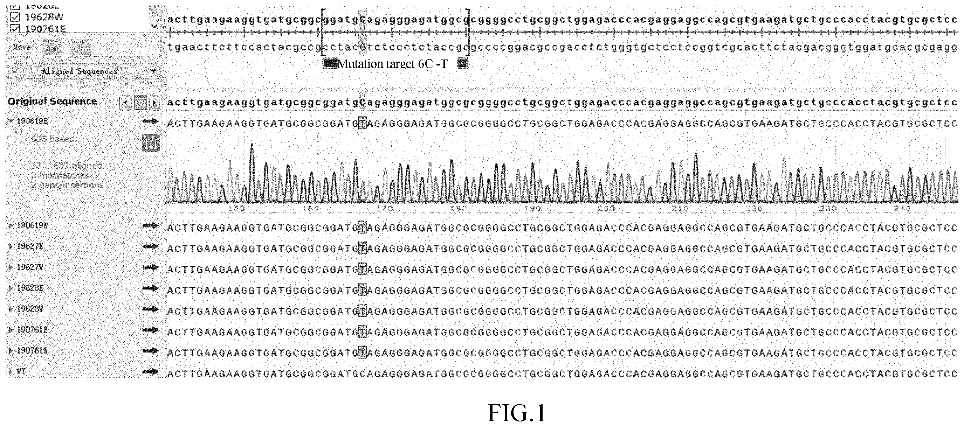

FIG. 1 shows the alignment of the sequences comprising 107 nucleotides around the mutation site of four point-mutation dogs and one wild-type dog. In FIG. 1, "W" refers to tail cells, "E" refers to ear cells and "WT" refers to the cells of the wild-type dog. It can be seen that all dogs 190619, 190627, 19062 and 190761 have point mutation as compared with the wild-type(WT) dog. The WT dog is not littermate and is of wild type.

### (a) Dog 190619:

The somatic cells of dog 190619 were classified as beagle skin fibroblasts GCK-KO-190619 comprising a point mutation in GCK gene, and were deposited in China General Microbiological Culture Collection Center (CGMCC) located at No. 3, Yard 1, Beichen West Road, Chaoyang District, Beijing 100101, on July 23, 2019, under CGMCC deposit No. 18305.

In particular, the wild-type dog GCK gene comprises the following sequence around the target site of exon 2:
5'-GGATGCAGAGGGAGATGGCG-3' (SEQ ID NO: 1).

The corresponding sequence comprising point mutation of dog 190619 comprises the following sequence:
5'-GGATGTAGAGGGAGATGGCG-3' (SEQ ID NO:2).

The mutant sequence can be determined by PCR using identification primers GCK-CBE-S2-F and GCK-CBE-S2-R, and recovered for sequencing:
GCK-CBE-S2-F: GGTCATTTGAGATGAGGGG (SEQ ID NO:4); and
GCK-CBE-S2-R: GAGGAGGAGAGGACGGAGT (SEQ ID NO:5).

(b) Table 3 indicates the daily blood glucose levels and body weights of point-mutation diabetic dog models 190619, 190627, 190628 and 190761, and wild-type dogs 190625 and 190626.

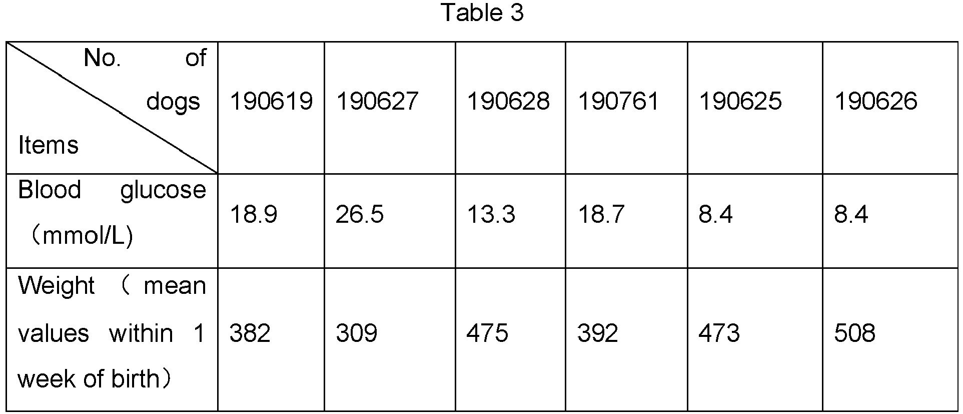

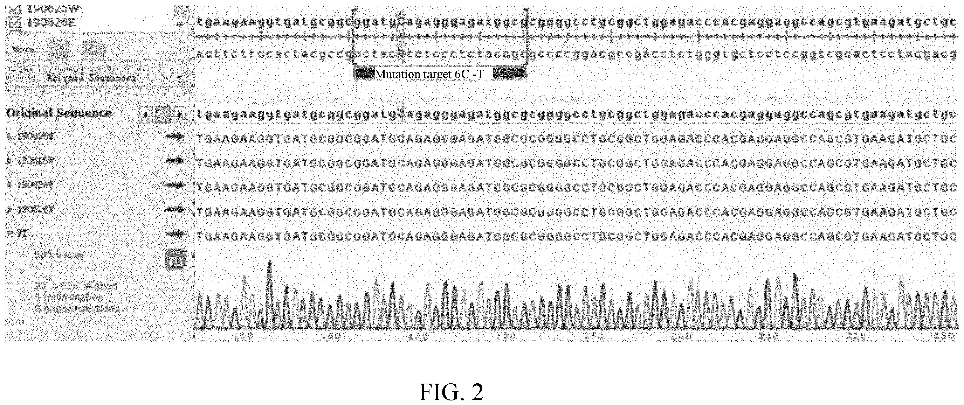

It can be seen from FIGS. 1 and 2 that, flanking the point mutation site, GCK gene point mutation dogs 190619, 190627, 190628 and 190761 have the same gene sequence as those of wild-type dogs 190625 and 190626, which are littermates of 190627 and 190628, and wild-type (WT) non-littermate dog.

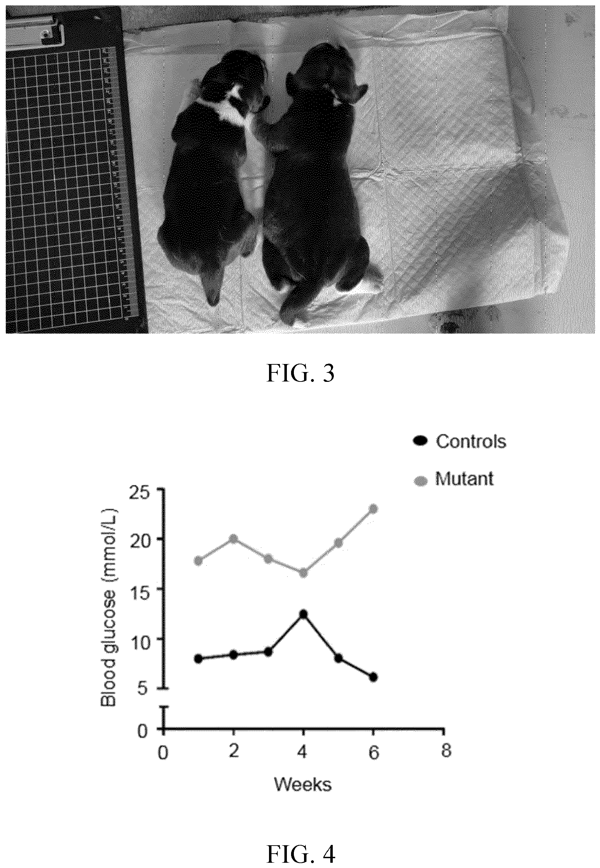

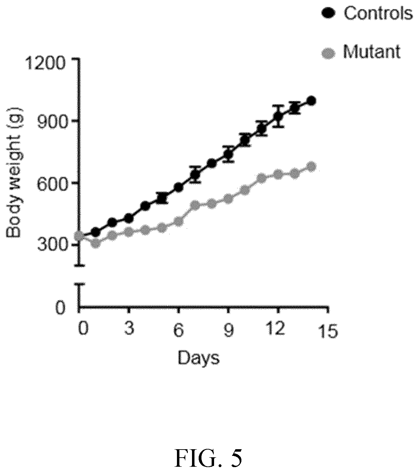

FIG. 4 shows that the blood glucose level of the mutant dog is significantly higher than that of wild-type littermate. FIG. 5 shows that the weight gain of the mutant dog is significantly slower than that of the wild-type littermate.

The above results indicate that the diabetic dog model 190627 prepared in the present disclosure comprising a point mutation in GCK gene exhibits diabetic phenotype, as compared with the wild-type littermates. Further, the four diabetic dog models 190619, 190627, 190628 and 190761 comprising a point mutation in GCK gene have the same genotype. Thus, it can conclude that the present disclosure has successfully prepared diabetic dog models point mutation in GCK gene.

## Claims

1. A method for preparing a diabetic dog model, comprising the following steps:
(1) obtaining a dog fertilized egg cell, which comprises a point mutation in GCK gene, for a diabetic dog model by means of gene editing; and
(2) transplanting the dog fertilized egg cell into one fallopian tube of a female dog, in which both fallopian tubes have been flushed, to prepare a diabetic dog model comprising a point mutation in GCK gene.

2. The method according to claim 1, wherein the gene editing in step (1) comprises BE3, CRISPR/Cas9, TALEN and ZFN.

3. A method for preparing a diabetic dog model comprising a point mutation in GCK gene, comprising the following steps:
(1) determining a targeting site directed to exon 2 based on the sequence of dog GCK gene;
(2) synthesizing an sgRNA sequence based on the targeting site determined in step (1), and ligating the synthesized sgRNA sequence into a backbone vector to construct an sgRNA targeting vector;
(3) obtaining sgRNA with *in vitro* transcription of the sgRNA targeting vector, and obtaining BE3 mRNA with *in vitro* transcription;
(4) mixing the sgRNA and BE3 mRNA obtained in step (3), and intracytoplasmically injecting into a dog fertilized egg cell; and
(5) trnsplanting the dog fertilized egg cell into one fallopian tube of a female dog, in which both fallopian tubes have been flushed, to prepare a diabetic dog model comprising a point mutation in GCK gene.

4. The method according to claim 3, further comprising: determining the targeting site directed to the exon 2 of GCK gene, and modifying the exon 2 with insertion, deletion, substitution and/or addition,
preferably, modifying the exon 2 with point mutation.

5. The method according to claim 4, wherein one target site in GCK gene is selected and comprises a sequence of 5'-GGATGCAGAGGGAGATGGCG-3' (SEQ ID NO: 1).

6. The method according to claim 4, wherein the sgRNA sequence synthesized based on the targeting site, and its complementary sequence comprise, respectively:
F: CACCGggatgcagagggagatggcg (SEQ ID NO: 8); and
R: AAACcgccatctccctctgcatccC (SEQ ID NO: 9).

7. The method according to claim 3, wherein in step (5), the dog fertilized egg cell is transplanted into one fallopian tube with less bleeding of the female dog, in which both fallopian tubes have been flushed.

8. A method for preparing a diabetic dog model comprising a point mutation in GCK gene, comprising the following steps:
(1) determining a targeting site directed to exon 2 based on the sequence of dog GCK gene;
(2) synthesizing an sgRNA sequence based on the targeting site determined in step (1), and ligating the synthesized sgRNA sequence into a backbone vector to construct an sgRNA targeting vector;
(3) obtaining sgRNA with *in vitro* transcription of the sgRNA targeting vector, and obtaining BE3 mRNA with *in vitro* transcription;
(4) mixing the sgRNA and BE3 mRNA obtained in step (3) and intracytoplasmically injecting into a dog somatic cell, and transplanting the nucleus of the dog somatic cell into a dog enucleated egg cell; and
(5) transplanting the dog enucleated egg cell into one fallopian tube of a female dog, in which both fallopian tubes have been flushed, to prepare a diabetic dog model comprising a point mutation in GCK gene.

9. A targeting vector for point mutation of dog GCK gene, comprising:
an sgRNA sequence, which is designed based on a targeting site directed to an exon, preferably exon 2, of dog GCK gene; and
a backbone sequence.

10. The targeting vector for point mutation of dog GCK gene according to claim 9, wherein the sgRNA sequence and its complementary sequence comprise, respectively:
F: CACCGggatgcagagggagatggcg (SEQ ID NO: 8) and
R: AAACcgccatctccctctgcatccC (SEQ ID NO:9).

11. A somatic cell, tissue or organ from the diabetic dog model comprising a point mutation in GCK gene obtained by the method of any one of claims 1 to 8.

12. The somatic cell, tissue or organ according to claim 11, which comprises a sequence as follows:
5'-GGATGTAGAGGGAGATGGCG-3' (SEQ ID NO: 2).

13. The somatic cell, tissue or organ according to claim 11, which comprises a sequence as follows:

GCAGAGCAGATCCTGGCGGACTTCCAGATGCAGGAGGCGGACTTGAAGAAGGT
GATGCGGCGGATGTAGAGGGAGATGGCGCGGGGCCTGCGGCTGGAGACCCAC
GAGGAGGCCAGCGTGAAGATGCTGCCCACCTACGTGCGCTCCACCCCAGAAG
GCTCAG (SEQ ID NO: 3).

14. The somatic cell, tissue or organ according to claim 11, which comprises a sequence as follows:

GAGCTACAAAGGGGGAGCTGCCACCTGCACCCCCCAGCATGCCTCGGCCACT
GCATTTGGGGGCTGCTCTTGGAACTCTGCTGCTCTAACTTTTCAACTGGTCCTA

ACTGGTCATTTGAGATGAGGGGTCATCTTGGGCCCAGGCCAGAGGGCAAAGGA
CACTCCCGGTGTGTGCAGGGAGGGCCTGACTTGGCTGCCACAGGCCCCCAGC
CCACCCAGCTTCCCCCCACCCCGTGCAGGCAGAGCAGATCCTGGCGGACTTCC
AGATGCAGGAGGCGGACTTGAAGAAGGTGATGCGGCGGATGTAGAGGGAGAT
GGCGCGGGGCCTGCGGCTGGAGACCCACGAGGAGGCCAGCGTGAAGATGCT
GCCCACCTACGTGCGCTCCACCCCAGAAGGCTCAGGTACCGCGTCCCTGGGC
CCCAGCAGGGAAGGCCAGCCTAGGTCCTTGCAGCCATGGGCTGGTTCTCACCC
GAGCTCCGTGCCAGCCCCGCCCTGGCATCTTCACCGCCTTGGGGAAACTGGCT
TTGTGCATCTTGCAATAGGAAATTTGTTCTCAGGCTCAGCCCAATCCTGACCCTG
CGCTGTTTCACTAGGTCTTTCCTTGGAACCTGGGCTTGCAGTGGCTTCAGTCCA
AGGCAGCGGCTGCCCACTGAGCTCCCCAGTTCACCTTTGACCTCTTCCACAGTT
GACTCTGGGGTCCCCTGGTACCTGGGGTCAGCAGCCCCCTGTCCAGCTCCTCT
GATCCCCCCACTCCGTCCTCTCCTCCTCTTCCTGATCTTGGGGTCCTGCTGGGC
AGCACCCCGAGGCTAATGCACTTCTCACTGTGTCTGCAGAAGTCGGGGACTTC
CTCTCCCTGGACCTGGGCGGCACCAACTTCAG (SEQ ID NO:4).

15. The somatic cell according to any one of claims 11 to 14, wherein the somatic cell is classified as skin fibroblast GCK-KO-190619 of beagle, which is a diabetic dog model comprising a point mutation in GCK gene, and the somatic cell is deposited in China General Microbiological Culture Collection Center (CGMCC) located at No. 3, Yard 1, Beichen West Road, Chaoyang District, Beijing 100101, on August 19, 2019, under CGMCC deposit No. 18305.

16. A pair of primers for detecting a diabetic dog model comprising a point mutation in GCK gene, wherein the diabetic dog model comprises a genomic sequence including a sequence as shown by 5'-GGATGTAGA GGGAGATGGCG-3' (SEQ ID NO: 2), and the pair of primers is designed based on the following sequence:

GAGCTACAAAGGGGGAGCTGCCACCTGCACCCCCCAGCATGCCTCGGCCACT
GCATTTGGGGGCTGCTCTTGGAACTCTGCTGCTCTAACTTTTCAACTGGTCCTA
ACTGGTCATTTGAGATGAGGGGTCATCTTGGGCCCAGGCCAGAGGGCAAAGGA
CACTCCCGGTGTGTGCAGGGAGGGCCTGACTTGGCTGCCACAGGCCCCCAGC
CCACCCAGCTTCCCCCCACCCCGTGCAGGCAGAGCAGATCCTGGCGGACTTCC
AGATGCAGGAGGCGGACTTGAAGAAGGTGATGCGGCGGATGTAGAGGGAGAT

GGCGCGGGGCCTGCGGCTGGAGACCCACGAGGAGGCCAGCGTGAAGATGCT
GCCCACCTACGTGCGCTCCACCCCAGAAGGCTCAGGTACCGCGTCCCTGGGC
CCCAGCAGGGAAGGCCAGCCTAGGTCCTTGCAGCCATGGGCTGGTTCTCACCC
GAGCTCCGTGCCAGCCCCGCCCTGGCATCTTCACCGCCTTGGGGAAACTGGCT
TTGTGCATCTTGCAATAGGAAATTTGTTCTCAGGCTCAGCCCAATCCTGACCCTG
CGCTGTTTCACTAGGTCTTTCCTTGGAACCTGGGCTTGCAGTGGCTTCAGTCCA
AGGCAGCGGCTGCCCACTGAGCTCCCCAGTTCACCTTTGACCTCTTCCACAGTT
GACTCTGGGGTCCCCTGGTACCTGGGGTCAGCAGCCCCCTGTCCAGCTCCTCT
GATCCCCCCACTCCGTCCTCTCCTCCTCTTCCTGATCTTGGGGTCCTGCTGGGC
AGCACCCCGAGGCTAATGCACTTCTCACTGTGTCTGCAGAAGTCGGGGACTTC
CTCTCCCTGGACCTGGGCGGCACCAACTTCAG (SEQ ID NO: 4).

17. A pair of primers for detecting a diabetic dog model comprising a point mutation in GCK gene, wherein the diabetic dog model comprises a genomic sequence including a sequence as shown by 5'-GGATGTAGA GGGAGATGGCG-3' (SEQ ID NO: 2), and the pair of primers is designed based on the following sequence:

GCAGAGCAGATCCTGGCGGACTTCCAGATGCAGGAGGCGGACTTGAAGAAGGT
GATGCGGCGGATGTAGAGGGAGATGGCGCGGGGCCTGCGGCTGGAGACCCAC
GAGGAGGCCAGCGTGAAGATGCTGCCCACCTACGTGCGCTCCACCCCAGAAG
GCTCAG (SEQ ID NO: 3).

18. The pair of primers according to claim 16 or 17, wherein the pair of primers comprises:
GCK-CBE-S2-F: 5' GGTCATTTGAGATGAGGGG 3' (SEQ ID NO: 5); and
GCK-CBE-S2-R: 5' GAGGAGGAGAGGACGGAGT 3' (SEQ ID NO: 6).

19. A kit for detecting a diabetic dog model comprising a point mutation in GCK gene, wherein the diabetic dog model comprises a genomic sequence including a sequence as shown by GGATGTAGAGGGAGATGGCG(SEQ ID NO: 2), and the kit comprises a pair of primers, which is designed based on the sequence as shown by SEQ ID NO: 4 or 3.

20. The kit according to claim 19, wherein the pair of primers comprises:
GCK-CBE-S2-F: 5' GGTCATTTGAGATGAGGGG 3' (SEQ ID NO: 5); and
GCK-CBE-S2-R: 5' GAGGAGGAGAGGACGGAGT 3' (SEQ ID NO: 6).

21. A diabetic dog model comprising a point mutation in GCK gene obtained by the method of any one of claims 1 to 8.
